# EUROPÄISCHE PATENTSCHRIFT

(11) **EP 1 634 544 B1**
(45) Veröffentlichungstag und Bekanntmachung des Hinweises auf die Patenterteilung: **08.10.2014**
(21) Anmeldenummer: 05018034.8
(22) Anmeldetag: 19.08.2005
(51) Int. Cl.: A61B 19/00, A61B 17/34

(54) **Halte- und Ablagevorrichtung für im wesentlichen zylindrische Instrumentenkörper aufweisende medizinische Instrumente**
Holding device for medical instruments with substantially cylindrical body
Dispositif de support pour instruments médicaux avec corps essentiellement cylindrique

(30) Priorität: 11.09.2004 DE 102004043982
(43) Veröffentlichungstag der Anmeldung: 15.03.2006
(73) Patentinhaber: Karl Storz GmbH & Co. KG, 78532 Tuttlingen (DE)
(72) Erfinder: Oberländer, Martin, 78532 Tuttlingen (DE); Sauer, Michael, 78532 Tuttlingen (DE); Schmidberger, Jochen, 72355 Schörzingen (DE)
(74) Vertreter: Hofmeister, Frank

(56) Entgegenhaltungen:
- EP-A- 0 426 407
- EP-A- 0 648 470
- NL-C2- 1 015 663
- US-A- 5 263 939
- US-A- 5 366 446
- US-A- 5 380 302
- US-A- 5 441 042
- US-A- 5 505 714
- US-A- 5 944 696
- US-B1- 6 228 059
- US-B1- 6 428 514

## Beschreibung

Die Erfindung betrifft eine Vorrichtung zur Ablage und Bereithaltung medizinischer Instrumente für endoskopische Untersuchungen, mit einem im wesentlichen zylindrischen Instrumentenkörper, mit einem beispielsweise an einem Operationstisch festlegbaren Grundkörper und mit einer in den Grundkörper einsetzbaren Instrumentenaufnahme, in der der Instrumentenkörper klemmend festlegbar ist.

Derartige Halte- und Ablagevorrichtung sind beispielsweise an Operationstischen angeordnet, um dem Operateur während der Operation ein sicheres Ablegen und wieder Ergreifen seiner Instrumente, beispielsweise eines Laparoskops oder Endoskops, zu ermöglichen, ohne hierfür OP-Personal zu benötigen. Wichtig bei diesen Haltevorrichtungen ist, dass diese das aufzunehmende medizinische Instrument sicher und möglichst lagestabil halten, um insbesondere ein Herunterfallen des Instruments zu verhindern.

Eine aus der Praxis bekannte Halte- und Ablagevorrichtung weist hierzu eine Instrumentenaufnahme auf, in der der Instrumentenkörper des zu haltenden medizinischen Instruments klemmend festlegbar ist, wobei das klemmende Halten über eine Spannschraube bewirkt wird, die nach mehreren Umdrehungen die Instrumentenaufnahme so verengt, dass der Instrumentenkörper sicher gehalten wird. Diese bekannte Halte- und Ablagevorrichtung gewährleistet zwar einen sicheren Halt des aufzunehmenden medizinischen Instruments, jedoch ist deren Bedienung für den Operateur umständlich, da das Lösen der Spannschraube einerseits Zeit kostet und andererseits in der Regel nur mit zwei Händen zu bewerkstelligen ist.

Aus der US-Patent 5 944 696 ist eine drehbare Halterung für medizinische Rohre bekannt, wie sie benutzt wird, um medizinische Rohrleitungen einem Patienten zuzuführen. Diese bekannte Vorrichtung besteht im Wesentlichen aus einer hülsenförmigen Aufnahme, in der das zu haltende Rohr verrastend festlegbar ist. Um eine für den Patienten angenehme Lagerung der zum Patienten führenden, in der Aufnahme gelagerten Rohrleitung zu gewährleisten, ist die hülsenförmige Aufnahme über einen radial abstehenden Stift verdreh- und verkippbar in einer Bodenplatte gelagert.

Weiterhin ist aus der US-Patent 5 505 714 eine Katheter-Kompressions-Klemme bekannt, die dazu dient, einen Katheter klemmend zu ergreifen und lagesicher in der richtigen Position im Patienten zu fixieren.

Dieser bekannte Klemmmechanismus besteht im Wesentlichen aus einer Aufnahme, in die der zu haltende Katheter in Längsrichtung einfügbar ist. Ein Teilbereich der Aufnahme ist als aus vier Haltearmen bestehende Spannhülse ausgebildet. Zum Festlegen des in der Aufnahme angeordneten Katheters sind die Haltearme über eine auf die Spannhülse aufsetzbare Spannschraube radial nach innen drückbar. In der Spannhülse ist ein hülsenförmiges elastisches Halteelement angeordnet, dass durch die radial nach innen gedrückten Haltearme ebenfalls radial nach innen verformt wird, bis es vollflächig an der Mantelfläche des zu haltenden Katheters anliegt.

Aus dem US-Patent 5 236 939 ist eine manuelle Klemmvorrichtung für in der Laparoskopie verwendete Kanülen bekannt. Diese bekannte Klemmvorrichtung besteht im Wesentlichen aus einer U-förmigen Klemme aus einem elastischen Material sowie einem in die U-förmige Klemme einsetzbaren, mit einer geschlitzten Durchgangsöffnung versehenen Einsatz zur Aufnahme der zu haltenden Kanüle. Zum Festlegen der Kanüle in dem Einsatz werden die parallelen Schenkel der U-förmigen Klemme manuell zusammengedrückt, bis der ebenfalls aus einem verfombaren Material bestehende Einsatz die eingesetzte Kanüle klemmend umschließt. Auch dieser geschlitzte Einsatz liegt in der Klemmstellung nahezu vollflächig an der Mantelfläche der zu haltenden Kanüle an.

Davon ausgehend liegt der Erfindung die **Aufgabe** zugrunde, eine Halte- und Ablagevorrichtung für medizinische Instrumente mit einem im Wesentlichen zylindrischen Instrumentenkörper zu schaffen, die bei einfachem Aufbau schnell und sicher zu betätigen ist und das Auftreten des Stick-Slip-Effekts vermeidet.

Die **Lösung** dieser Aufgabenstellung ist erfindungsgemäß dadurch gekennzeichnet, dass die Instrumentenaufnahme eine mindestens zwei Haltearme aufweisende ringförmige Spannhülse sowie eine mit der Spannhülse zusammenwirkende Spannmutter umfasst, wobei in der Spannhülse ein aus einem elastisch verformbaren Material bestehendes, ringförmiges Halteelement zur Aufnahme des Instrumentenkörpers angeordnet ist, dessen dem aufzunehmenden Instrumentenkörper zugewandte Innenfläche zur Minimierung der Kontaktfläche mit dem Instrumentenkörper gezahnt so ausgebildet ist, dass diese in der Klemmstellung nur im Bereich der Zähne am Instrumentenkörper anliegt.

Die erfindungsgemäße Ausstattung der Instrumentenaufnahme mit der Spannhülse sowie der mit der Spannhülse zusammenwirkenden Spannmutter ermöglicht einen einfachen und kostengünstigen Aufbau der Halte- und Ablagevorrichtung.

Durch das in die Spannhülse einsetzbare, aus einem elastisch verformbaren Material bestehende ringförmige Halteelement zur Aufnahme des Instrumentenkörpers wird der sichere und schonende Halt des Instrumentenkörpers in der Instrumentenaufnahme verbessert.

Um einerseits das Einsetzen des Instrumentenkörpers in das elastisch verformbare Halteelement der Instrumentenaufnahme zu erleichtern und andererseits das Auftreten sogenannter "Knarzgeräusche" aufgrund des Stick-Slip-Effekts zu vermeiden, liegt die dem aufzunehmenden Instrumentenkörper zugewandte Innenfläche des elastischen Halteelements in der Klemmstellung nur bereichsweise am Instrumentenkörper an, wozu zur Minimierung der Kontaktfläche die dem aufzunehmenden Instrumentenkörper zugewandte Innenfläche des elastischen Halteelements gezahnt ausgebildet ist. Da der Stick-Slip-Effekt durch ein Wechselspiel von Haft- und Gleitreibung zweier entlang einander gleitender Materialien auftritt, stellt die Minimierung der gegenseitigen Kontaktflächen eine geeignete Maßnahme zur Vermeidung dieser "Knarzgeräusche" dar.

Gemäß einer praktischen Ausführungsform der Erfindung wird vorgeschlagen, dass die Haltearme der Spannhülse über an der Spannmutter ausgebildete Spannelemente im wesentlichen radial verformbar sind. Das klemmende Halten des Instrumentenkörpers in der Instrumentenaufnahme erfolgt dabei durch das radial nach innen Verformen der Haltearme der Spannzange, wenn die Spannelemente der Spannmutter gegen die Haltearme anlaufen. Da die Haltearme der Spannzange durch jedes Spannelement klemmend nach innen gedrückt werden, bilden diese zusammenwirkenden Spannwerkzeuge einen Schnellspannverschluss bei dem es keiner vollständigen Umdrehung der Spannmutter bedarf, um ein in die Haltevorrichtung eingestecktes medizinisches Instrument sicher festzulegen und wieder zu lösen.

Vorzugsweise weist die erfindungsgemäße Spannhülse vier gleichmäßig um den Umfang der ringförmigen Spannhülse angeordnete Haltearme auf, die über vier an der Spannmutter ausgebildete, als Spannelemente dienende Nocken verformbar sind. Bei dieser Ausführungsform bedarf es lediglich einer viertel Umdrehung der Spannmutter, um die Haltevorrichtung von der Offenstellung in die Klemmstellung bzw. von der Klemmstellung in die Offenstellung zu überführen.

Weitere Merkmale und Vorteile der Erfindung ergeben sich anhand der zugehörigen Zeichnung, in der ein Ausführungsbeispiel einer erfindungsgemäßen Halte- und Ablagevorrichtung für im wesentlichen zylindrische Instrumentenkörper aufweisende medizinische Instrumente beispielhaft dargestellt ist. In der Zeichnung zeigt:
- Fig. 1: eine Explosionszeichnung einer erfindungsgemäßen Halte- und Ablagevorrichtung für medizinische Instrumente mit einem im wesentlichen zylindrischen Instrumentenkörper;
- Fig. 2: einen Längsschnitt durch die Haltevorrichtung gemäß Fig. 1 im zusammengebauten Zustand;
- Fig. 3: eine ausschnittweise Draufsicht auf die Haltevorrichtung gemäß Fig. 2, die Offenstellung darstellend und
- Fig. 4: eine Draufsicht gemäß Fig. 3, jedoch die Klemmstellung darstellend.

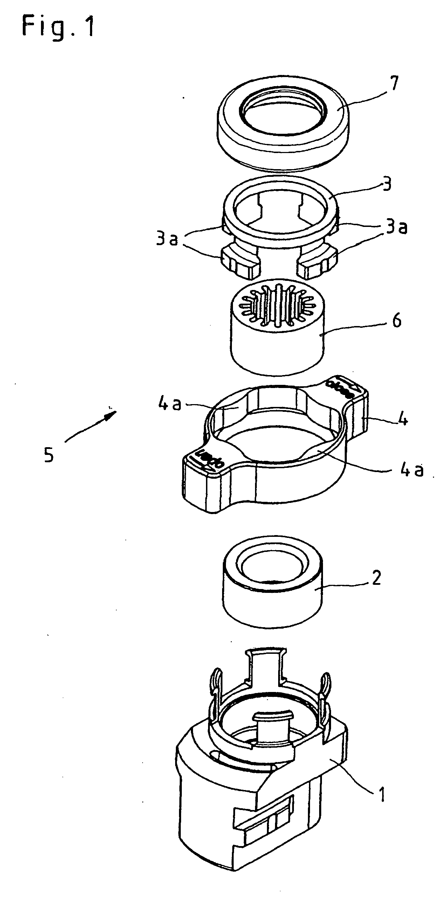

Die in den Abbildungen dargestellte Halte- und Ablagevorrichtung für im wesentlichen zylindrische Instrumentenkörper aufweisende medizinische Instrumente besteht, wie insbesondere aus der Explosionszeichnung gemäß Fig. 1 ersichtlich, aus einem beispielsweise an einem Operationstisch festlegbaren Grundkörper 1 sowie einem in den Grundkörper 1 einsetzbaren Führungsring 2, einer vier Haltearme 3a aufweisenden ringförmigen Spannhülse 3 und einer auf die Spannhülse 3 aufsetzbaren Spannmutter 4, die zusammen eine Instrumentenaufnahme 5 bilden, sowie einem in die Spannhülse 3 der Instrumentenaufnahme 5 einsetzbaren ringförmigen Halteelement 6 und einem oberen Abdeckring 7.

Alternativ zum Festlegung des Grundkörpers 1 der Halte- und Ablagevorrichtung an einem Operationstisch ist es selbstverständlich auch möglich, den Grundkörper 1 an einem anderen Gegenstand, beispielsweise einem Gerätetisch oder einem separaten Ständer festzulegen.

Insbesondere aus den Abbildungen Fig. 1, 3 und 4 ergeben sich der Aufbau und die Arbeitsweise der aus der Spannhülse 3 und der Spannmutter 4 bestehenden Instrumentenaufnahme 5, die zur Aufnahme und zum Fixieren des Instrumentenkörpers eines in die Haltevorrichtung eingesteckten medizinischen Instruments, beispielsweise eines Laparoskops oder Endoskops, dient.

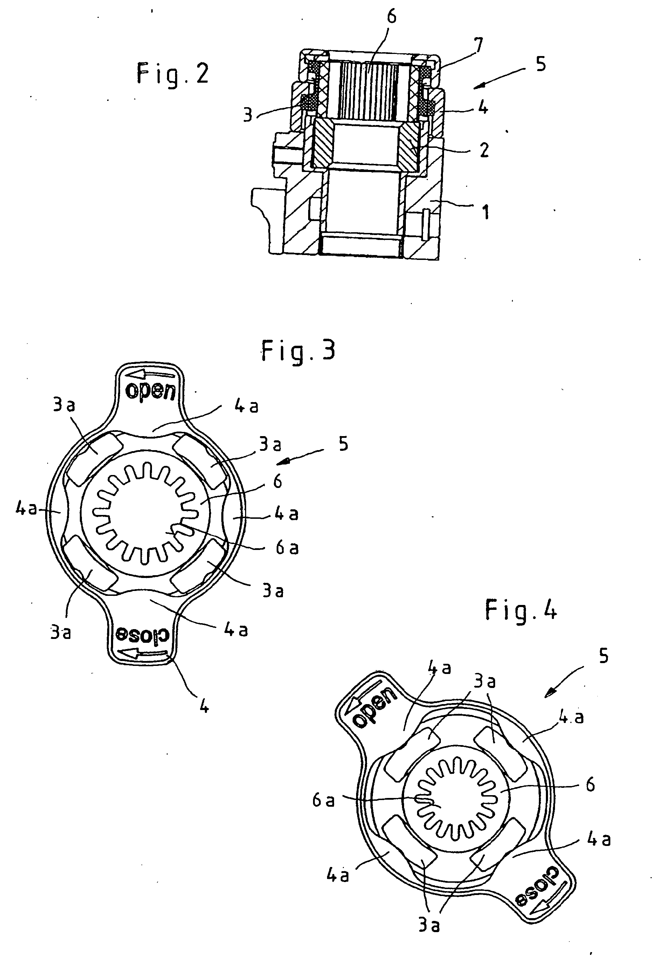

Wie insbesondere aus den Draufsichten gemäß Fig. 3 und 4 ersichtlich, weist die Spannmutter 4 auf ihrer der Spannhülse 3 zugewandten Innenseite vier als Nocken 4a ausgebildete Spannelemente auf, die das klemmende Zusammenwirken von Spannhülse 3 und Spannmutter 4 bewirken.

Das klemmende Ergreifen eines in die Instrumentenaufnahme 5 eingesetzten medizinischen Instruments erfolgt dabei wie folgt:
In der in Fig. 3 dargestellten Offenstellung der Instrumentenaufnahme 5 sind die Nocken 4a der die Spannhülse 3 koaxial umgebenden Spannmutter 4 jeweils abwechselnd zwischen den Haltearmen 3a der Spannhülse 3 angeordnet. Sobald die Spannmutter 4 um eine viertel Umdrehung nach rechts oder links gedreht wird, laufen die Nocken 4a der Spannmutter 4 gegen die Haltearme 3a der Spannhülse 3 an und drücken diese im wesentlichen radial nach innen, so dass der Aufnahmeraum im Inneren der Spannhülse im Durchmesser verkleinert wird, wie dies die in Abbildung Fig. 4 dargestellte Klemmstellung zeigt.

Ein in diesem Aufnahmeraum angeordneter Instrumentenkörper wird in der Klemmstellung von den radial nach innen verformten Haltearmen 3a der Spannhülse 3 klemmend ergriffen und in seiner Lage fixiert.

Zum Öffnen der Instrumentenaufnahme 5 ist es lediglich notwendig, die Spannmutter 4 wieder um eine viertel Umdrehung nach rechts oder links zu drehen, bis die Nocken 4a der Spannmutter 4 nicht mehr in Eingriff mit den Haltearmen 3a der Spannhülse 3 stehen.

Auch wenn bei der dargestellten Ausführungsform einer Haltevorrichtung auf der Spannmutter 4 bevorzugte Drehrichtungen zum Öffnen und Schließen der Instrumentenaufnahme 5 angegeben sind, besteht aufgrund der konstruktiven Ausgestaltung die prinzipielle Möglichkeit, die Spannmutter 4 zum Öffnen und Schließen der Instrumentenaufnahme 5 jeweils in beide Richtungen zu drehen.

Ebenso ist es selbstverständlich möglich, die Spannhülse 3 mit mehr oder weniger vielen Haltearmen 3a auszustatten. Um ein einfaches und schnelles Betätigen der Instrumentenaufnahme 5 zu gewährleisten, ist es vorteilhaft, wenn die Anzahl der Nocken 4a der Spannmutter 4 der Anzahl der Haltearme 3a der Spannhülse 3 entspricht.

Wie weiterhin aus den Abbildungen ersichtlich, weist die Instrumentenaufnahme 5 weiterhin ein in die Spannhülse 3 eingesetztes ringförmiges Halteelement 6 auf, das aus einem elastischen Material hergestellt dazu dient, einen sicheren und schonenden Halt des in die Instrumentenaufnahme 5 eingesetzten Instrumentenkörpers zu gewährleisten. Im Gegensatz zu den radial nach innen gebogenen, nur punktuell angreifenden Haltearmen 3a umschließt das Halteelement 6 den gesamten Aufnahmeraum und somit auch den gesamten Instrumentenkörper.

Um das Einsetzen des Instrumentenkörpers in das elastisch verformbare, vorzugsweise aus einem gummiartigen Material gefertigte Halteelement 6 zu erleichtern und das Auftreten sogenannter "Knarzgeräusche" aufgrund des Stick-Slip-Effekts zu vermeiden, liegt die dem aufzunehmenden Instrumentenkörper zugewandte Innenfläche 6a des elastischen Halteelements 6 in der Klemmstellung nur bereichsweise am Instrumentenkörper an. Bei der dargestellten Ausführungsform ist die Innenfläche 6a des Halteelements 6 zu diesem Zweck gezahnt ausgebildet.

Bei dem Stick-Slip-Effekt - auch Ruckgleiten genannt - geraten zwei Materialien angeregt durch Relativbewegungen zueinander in ein Wechselspiel zwischen Haft-und Gleitreibung. Dieses Hin und Her zwischen Gleiten und Verhaken ist die Ursache für diese "Knarzgeräusche". Durch die Minimierung der Kontaktflächen zwischen dem Halteelement 6 einerseits und dem Instrumentenkörper andererseits wird das Auftreten des Stick-Slip-Effekts beim Einstecken und Herausziehen des Instrumentenkörpers aus der Instrumentenaufnahme 5 vermieden.

Eine wie beschrieben ausgebildete Halte- und Ablagevorrichtung für im wesentlichen zylindrische Instrumentenkörper aufweisende medizinische Instrumente zeichnet sich dadurch aus, dass die Instrumentenaufnahme 5 als Schnellspannverschluss ausgebildet ist. Diese Ausgestaltung ermöglicht dem Operateur ein einfaches und sicheres Bedienen der Haltevorrichtung auch mit nur einer Hand.

### Bezupszeichenliste

- 1: Grundkörper
- 2: Führungsring
- 3: Spannhülse
- 3a: Haltearm
- 4: Spannmutter
- 4a: Nocken
- 5: Instrumentenaufnahme
- 6: Halteelement
- 6a: Innenfläche
- 7: Abdeckring

## Patentansprüche

1. Vorrichtung zur Ablage und Bereithaltung medizinischer Instrumente für endoskopische Untersuchungen, mit einem im wesentlichen zylindrischen Instrumentenkörper, mit einem beispielsweise an einem Operationstisch festlegbaren Grundkörper (1) und mit einer in den Grundkörper (1) einsetzbaren Instrumentenaufnahme (5), in der der Instrumentenkörper klemmend festlegbar ist,
**dadurch gekennzeichnet,**
**dass** die Instrumentenaufnahme (5) eine mindestens zwei Haltearme (3a) aufweisende ringförmige Spannhülse (3) sowie eine mit der Spannhülse (3) zusammenwirkende Spannmutter (4) umfasst, wobei in der Spannhülse (3) ein aus einem elastisch verformbaren Material bestehendes, ringförmiges Halteelement (6) zur Aufnahme des Instrumentenkörpers angeordnet ist, dessen dem aufzunehmenden Instrumentenkörper zugewandte Innenfläche (6a) zur Minimierung der Kontaktfläche mit dem Instrumentenkörper gezahnt so ausgebildet ist, dass diese in der Klemmstellung nur im Bereich der Zähne am Instrumentenkörper anliegt.

2. Vorrichtung nach Anspruch 1, **dadurch gekennzeichnet, dass** die Haltearme (3a) der Spannhülse (3) über an der Spannmutter (4) ausgebildete Spannelemente im wesentlichen radial verformbar sind.

3. Vorrichtung nach Anspruch 2, **dadurch gekennzeichnet, dass** die Spannhülse (3) vier gleichmäßig um den Umfang der ringförmigen Spannhülse (3) angeordnete Haltearme (3a) aufweist, die über vier an der Spannmutter (4) ausgebildete, als Spannelemente dienende Nocken (4a) verformbar sind.

## Claims

1. Device for storing and presenting medical instruments for endoscopic examinations, with a substantially cylindrical instrument body, with a base body (1) which can be immobilized on an operating table, for example, and with an instrument receiver (5) which can be inserted in the base body (1), and in which the instrument body can be immobilized by clamping,
**characterized in**
**that** the instrument receiver (5) includes an annular clamping sleeve (3) comprising at least two supporting arms (3a), as well as a clamping nut (4) which works together with the clamping sleeve (3), wherein, in the clamping sleeve (3), an annular supporting element (6) consisting of a resiliently deformable material is arranged for receiving the instrument body, the inner surface (6a) of said supporting element, which faces the instrument body to be received, being formed with toothing to minimize the contact surface with the instrument body. in such a manner that said contact surface in the clamping position rests only in the area of the teeth against the instrument body.

2. Device according to Claim 1, **characterized in that** the supporting arms (3a) of the clamping sleeve (3) are substantially radially deformable by means of clamping elements formed on the clamping nut (4).

3. Device according to Claim 2, **characterized in that** the clamping sleeve (3) comprises four supporting arms (3a) which are arranged regularly around the circumference of the annular clamping sleeve (3), and which are deformable by means of four cams (4a) formed on the clamping nut (4) and used as clamping elements.

## Revendications

1. Dispositif pour supporter et mettre à disposition des instruments médicaux destinés à des investigations endoscopiques et présentant un corps d'instrument sensiblement cylindrique, le dispositif comprenant un corps de base (1), qui peut, par exemple, être fixé à une table d'opération, et un logement d'accueil d'instrument (5), qui peut être inséré dans le corps de base (1) et dans lequel le corps d'instrument peut être fixé par serrage,
**caractérisé**
**en ce que** le logement d'accueil d'instrument (5) comprend une douille de serrage (3) de forme annulaire présentant au moins deux bras de maintien (3a), ainsi qu'un écrou de serrage (4) interagissant avec la douille de serrage (3), un élément de maintien (6) de forme annulaire, destiné à recevoir le corps d'instrument et réalisé en un matériau qui peut être déformé de manière élastique, étant agencé dans la douille de serrage (3), et la surface intérieure (6a) de cet élément de maintien, qui est dirigée vers le corps d'instrument et doit recevoir le corps d'instrument, étant d'une configuration dentée telle, que dans la position de serrage, elle s'applique contre le corps d'instrument uniquement dans la région des dents, en vue de minimiser la surface de contact avec le corps d'instrument.

2. Dispositif selon la revendication 1, **caractérisé en ce que** les bras de maintien (3a) de la douille de serrage (3) peuvent être déformés de manière essentiellement radiale par l'intermédiaire d'éléments de serrage formés sur l'écrou de serrage (4).

3. Dispositif selon la revendication 2, **caractérisé en ce que** la douille de serrage (3) présente quatre bras de maintien (3a), qui sont agencés de manière uniformément répartie sur la périphérie de la douille de serrage (3) de forme annulaire, et qui peuvent être déformés par quatre cames (4a) formées sur l'écrou de serrage (4) et servant d'éléments de serrage.
